# EUROPEAN PATENT APPLICATION

(11) **EP 1 435 354 A1**
(43) Date of publication of application: **07.07.2004**
(21) Application number: 02800787.0
(22) Date of filing: 08.10.2002
(51) Int. Cl.: C07D 307/60, C08G 63/78

(54) **SUCCINIC ANHYDRIDE, PROCESS FOR PREPARATION OF THE SAME AND USE THEREOF**

(30) Priority: 09.10.2001 JP 2001311939
(71) Applicant: Nippon Shokubai Co., Ltd., Osaka-shi, Osaka 541-0043 (JP)
(72) Inventor: ITO, Rie, Moriyama-shi, Shiga 524-0042 (JP); SASAKI, Masamitsu, Himeji-shi, Hyogo 671-1143 (JP); SHIROSHIMA, Masahiro, Himeji-shi, Hyogo 671-1242 (JP); KAKIMOTO, Yukihiko, Yokohama-shi, Kanagawa 245-0007 (JP); MATSUMOTO, Koji, Himeji-shi, Hyogo 671-1146 (JP)
(74) Representative: Henkel, Feiler & Hänzel
(86) International application number: PCT/JP2002/010426
(87) International publication number: WO 2003/031426

(57) **Abstract**

An object of the present invention is to provide: succinic anhydride that little becomes colored in a melted state; a production process for such succinic anhydride; and a production process for a polyester, by which there can be obtained a polyester of an extremely reduced color. As means of achieving such an object, the succinic anhydride is characterized by having a γ-butyrolactone content of not more than 800 ppm and a dilactone content of not more than 500 ppm. The production process for the succinic anhydride is characterized in that: the hydrogenation reaction is carried out in the presence of a catalyst in the temperature range of 120 to 160 °C under a hydrogen pressure of 0.1 to 2.0 MPa; and the distillation is carried out in such a manner that the bottom temperature will be within the temperature range of 125 to 200 °C by reducing the pressure. The production process for the polyester is characterized in that the aforementioned succinic anhydride is used as an acid component.

## Description

### TECHNICAL FIELD

The present invention relates to succinic anhydride and its production process and uses. More particularly, the present invention relates to: succinic anhydride that is suitable as a raw material for production of polyesters; a process for obtaining the aforementioned succinic anhydride, which comprises the steps of carrying out a hydrogenation reaction of maleic anhydride as a raw material and then purifying the resultant succinic anhydride by distillation; and a production process for a polyester, by which there can be obtained a polyester of a reduced color.

### BACKGROUND ART

Succinic anhydride is useful in various fields and is widely used as such as a raw material for production of succinic acid, diesters of succinic acid, succinimides, polyesters (particularly, biodegradable polyesters (aliphatic polyesters)), and drugs.

As processes for obtaining the succinic anhydride, there have hitherto been known such as: processes that involve dehydrating succinic acid as a raw material (e.g. a process that involves heating the succinic acid together with phosphorus oxychloride in the temperature range of 100 to 120 °C, thereby dehydrating the succinic acid; a process that involves dehydrating the succinic acid with a water-azeotropic solvent or a hydrophilic solvent at a temperature of not higher than 100 °C; and a process that involves dehydrating the succinic acid alone at a temperature of not lower than 200 °C); and processes that involve carrying out such as hydrogenation of maleic anhydride as a raw material in the presence of a catalyst (JP-A-017707/1972 and British Patent No. 1,332,613). For example, when high-quality succinic anhydride is obtained on a small scale for such as reagent uses, the processes in which the succinic acid is used as the raw material are favorable and are, also in general, adopted. However, when the succinic anhydride is produced on an industrial scale such as in the case of using the succinic anhydride as various raw materials for production, then the processes in which the maleic anhydride is used as the raw material are favorable.

In the case where the succinic anhydride is obtained from the maleic anhydride, it is usually necessary to purify a reaction product (crude succinic anhydride) as obtained by carrying out the hydrogenation reaction of the raw maleic anhydride. As to the purification, distillation is generally carried out because the distillation has industrial advantages in point of such as operational convenience and facilities. In addition, various studies have hitherto been made to produce purified succinic anhydride excellent in product quality. However, when the purification is carried out by the distillation, there have been cases where the coloring, particularly, yellow coloring, occurs to the purified succinic anhydride in spite of success in the removal of the succinic acid. Such coloring remarkably occurs especially when the succinic anhydride is put in a melted state. For example, in the case where the above succinic anhydride is used in the melted state as a raw material for production of polyesters, there has been a problem of causing the remarkable coloring of the polyesters as obtained. In addition, such a problem of causing the coloring of the polyesters has been similarly problematic also in the case where the polyesters are produced from succinic anhydride that is commercially available as a reagent (Japanese Patent No. 3048316).

Accordingly, hitherto, succinate polyesters have generally been produced from the succinic acid. However, industrially, the succinic anhydride having a lower melting point than the succinic acid is easier to handle than the succinic acid. In addition, in the case of using the succinic acid as the raw material, it takes so long time to carry out its dehydration that there is also a problem such that the reaction time is longer and the productivity therefore tends to be inferior, when compared with the case of using the succinic anhydride as the raw material,. Accordingly, it is essentially desired to use the succinic anhydride as the raw material, and it is demanded to avoid the problem of the coloring in the case of using the succinic anhydride as the raw material.

### DISCLOSURE OF THE INVENTION

### OBJECT OF THE INVENTION

An object of the present invention is to provide: succinic anhydride that little becomes colored in a melted state; a production process for such succinic anhydride; and a production process for a polyester, by which there can be obtained a polyester of an extremely reduced color.

### SUMMARY OF THE INVENTION

The present inventors diligently studied to solve the above-mentioned problems.

As a result, when the present inventors examined conventional purified succinic anhydride, they have noticed the existence of 1,6-dioxaspiro[4,4]nonane-2,7-dione (which may hereinafter be referred to as dilactone) that is formed by dimerization of the succinic anhydride and thus contained therein as a by-product. And then the present inventors have discovered that it is a cause of the coloring of the purified succinic anhydride that the above dilactone is contained therein in a large amount. Furthermore, the present inventors have also found out that: the above dilactone is formed due to heating during the distillation; and besides, the formed dilactone is so difficult to remove by the distillation as to remain in the purified succinic anhydride to thus cause the coloring. Thus, the present inventors have repeated the study about distillation conditions under which the dilactone forms little. As a result, the present inventors have discovered that: if the column-bottom temperature of the distillation column is set so as to come within a definite range by reducing the pressure, then the formation of the dilactone can be suppressed.

However, as to such as uses for which the still less coloring is required (e.g. the case of using the succinic anhydride as a raw material for production of polyesters), the expected reduction alone of the coloring by suppressing the formation of the dilactone is insufficient. Therefore, the present inventors have presumed that there is some other coloring-causing factor besides the dilactone. Specifically, the reason therefor is as follows: even if conditions are defined to such an extent that only the concentration of the dilactone as considered to be the main cause of the coloring will be as low as possible and even if, as a result, there is obtained succinic anhydride of quality as high as possible, then the problem of the coloring is still not solved to not lower than a definite level, and besides, the burden merely increases in point of the productivity and costs; and, as a means of solving such problems at a stroke, the present inventors have conceived that it may still be necessary to direct their attention to something other than the dilactone. Thus, the present inventors have repeated various studies and trial and errors. As a result, the present inventors have directed their attention to γ-butyrolactone as contained as a by-product in conventional produced succinic anhydride similarly to the dilactone, and then the present inventors have discovered that, also as to this γ-butyrolactone similarly to the dilactone, it is a cause of the coloring that the γ-butyrolactone is still contained in a large amount in the purified succinic anhydride. In addition, the present inventors have further found out that: the γ-butyrolactone is easily formed when the hydrogenation reaction of the raw maleic anhydride further proceeds to excess during this reaction; and besides, the formed γ-butyrolactone is so difficult to remove by the subsequent distillation as to remain in the purified succinic anhydride to thus cause the coloring. Thus, the present inventors have repeated the study about conditions of the hydrogenation reaction under which the γ-butyrolactone forms little. As a result, the present inventors have discovered that: if the reaction temperature and the hydrogen pressure during the hydrogenation reaction are set so as to come within their respective definite ranges, then the formation of the γ-butyrolactone can be suppressed.

Accordingly, the present inventors have completed the present invention by confirming that: if the dilactone content and the γ-butyrolactone content are decreased to not higher than their respective definite amounts in the purified succinic anhydride in the above ways, then there is obtained succinic anhydride that less becomes colored even in a melted state, so that the above problems can be solved at a stroke. In addition, the present inventors have further completed the present invention by further confirming that: if the production process for succinic anhydride is a process in which the reaction conditions in the hydrogenation reaction and the distillation conditions are specified as mentioned above, then the above problems can be solved at a stroke. Moreover, the present inventors have further completed the present invention by further confirming that: if the polyester is produced from such succinic anhydride as aforementioned, then there can be obtained a polyester of an extremely reduced color, so that the above problems can be solved at a stroke.

That is to say, succinic anhydride, according to the present invention, is a raw material for production of polyesters and is obtained by a production process including the steps of: obtaining crude succinic anhydride by a hydrogenation reaction of maleic anhydride used as a raw material; and then purifying the crude succinic anhydride by distillation; with the succinic anhydride being characterized by having a γ-butyrolactone content of not more than 800 ppm and a dilactone content of not more than 500 ppm.

In addition, a production process for succinic anhydride, according to the present invention, comprises the steps of: obtaining crude succinic anhydride by a hydrogenation reaction of maleic anhydride used as a raw material; and then purifying the crude succinic anhydride by distillation; with the production process being characterized in that: the hydrogenation reaction is carried out in the presence of a catalyst in the temperature range of 120 to 160 °C under a hydrogen pressure of 0.1 to 2.0 MPa; and the distillation is carried out in such a manner that the bottom temperature will be within the temperature range of 125 to 200 °C by reducing the pressure.

Furthermore, a production process for a polyester, according to the present invention, comprises the step of carrying out a reaction between an acid component and an alcohol component to thereby produce the polyester; with the production process being characterized in that the aforementioned succinic anhydride according to the present invention is used as the acid component.

The succinic anhydride according to the present invention is succinic anhydride of which the coloring is more reduced by specifying the contents of both by-products, namely, the dilactone and the γ-butyrolactone. However, even if the content is high as to either one of these by-products, the content of the other is also suppressed to a low one. Therefore, as a result, it is also possible to render the coloring of the succinic anhydride lower than that of succinic anhydride in which the content of either one of the by-products is lowered as much as possible. Naturally, if both contents are sufficiently lowered as much as possible, the coloring can be suppressed remarkably. These effects relate also to the production process for succinic anhydride according to the present invention. If conditions of the hydrogenation reaction and of the distillation are fitly set on their respective appropriate levels and thus be combined together, then it is also possible to obtain succinic anhydride of a more reduced color due to so-called synergistic effects, and besides, it is also possible to obtain greatly advantageous effects in point of such as productivity and costs, when compared with succinic anhydride as obtained by limiting the conditions of only one of the hydrogenation reaction and the distillation to the ranges as favorable as possible.

### DETAILED DESCRIPTION OF THE INVENTION

Hereinafter, detailed descriptions are specifically given about the succinic anhydride, its production process, and the production process for a polyester according to the present invention. However, the scope of the present invention is not bound to these descriptions in any way. And other than the following illustrations can also be carried out appropriately within the scope not departing from the spirit of the present invention.

The succinic anhydride according to the present invention (which may hereinafter be referred to as the present invention succinic anhydride) is usable as a raw material for production of polyesters and is obtained by a production process including the steps of: obtaining crude succinic anhydride by a hydrogenation reaction of maleic anhydride used as a raw material; and then purifying the crude succinic anhydride by distillation.

The present invention succinic anhydride is favorably succinic anhydride including neither the γ-butyrolactone nor the dilactone that are by-products. However, in the case where the succinic anhydride includes them, the γ-butyrolactone content is not more than 800 ppm, favorably not more than 500 ppm, more favorably not more than 400 ppm, and the dilactone content is not more than 500 ppm, favorably not more than 300 ppm, more favorably not more than 100 ppm, still more favorably not more than 50 ppm. Hereupon, the above dilactone is specifically 1,6-dioxaspiro[4,4]nonane-2,7-dione. Such succinic anhydride, according to the present invention, can be obtained, for example, by the below-mentioned present invention production process for succinic anhydride.

In the case where the γ-butyrolactone content is more than 800 ppm in the present invention succinic anhydride, this γ-butyrolactone reacts with a trace of oxygen in a melted state to thereby easily form coloring-causing substances. Therefore, even if the coloring does not occur in the initial stage of the melting, the coloring becomes greater as the melting time becomes longer. In addition, the dilactone is one of the substances causing the coloring of the succinic anhydride. Therefore, the case where the dilactone content is more than 500 ppm results in causing the coloring in a melted state. Thus, such succinic anhydride, which has a γ-butyrolactone and/or dilactone content of more than the aforementioned ranges and becomes colored in a melted state, causes remarkable coloring also of the resultant polyesters when used as a raw material for production of the polyesters. Therefore, such succinic anhydride is not suitable as a raw material for production of the polyesters. Specifically, in the case where the succinic anhydride has a Yellow-Index (YI) of not less than 3, or in the case where the succinic anhydride displays a change of the Yellow-Index (ΔYI) of not less than 8 when the succinic anhydride is left in a melted state of 150 °C for 7 hours, then the resultant polyesters becomes remarkably colored in both cases. Accordingly, the present invention succinic anhydride, favorably, has a Yellow-Index (YI) of less than 3 and displays a change of the Yellow-Index (ΔYI) of less than 8 when the succinic anhydride is left in a melted state of 150 °C for 7 hours.

The production process for succinic anhydride according to the present invention (which may hereinafter be referred to as the present invention production process) is a process which comprises the steps of: obtaining crude succinic anhydride by a hydrogenation reaction of maleic anhydride used as a raw material; and then purifying the crude succinic anhydride by distillation; wherein: the hydrogenation reaction is carried out in the presence of a catalyst in the temperature range of 120 to 160 °C under a hydrogen pressure of 0.1 to 2.0 MPa; and thereafter the distillation is carried out in such a manner that the bottom temperature will be within the temperature range of 125 to 200 °C by reducing the pressure.

In the present invention production process, there is no especial limitation on the maleic anhydride used as the raw material. For example, the maleic anhydride will do if it is a product obtained by such as methods including the step of carrying out catalytic gas phase oxidation of benzene with air or a molecular-oxygen-containing gas by a fixed-bed process (refer to such as JP-B-054944/1991 and JP-B-019108/1963).

In the present invention production process, it is important that the reaction temperature is set in the range of 120 to 160 °C when the hydrogenation reaction of the raw maleic anhydride is carried out. This reaction temperature is more favorably set in the range of 120 to 150 °C, still more favorably 120 to 145 °C. In the case where the reaction temperature in the hydrogenation reaction is lower than 120 °C, then, because the melting point of the succinic anhydride being formed is close to 120 °C, the viscosity of the reaction liquid rises to slow the reaction rate. In the case where the reaction temperature is higher than 160 °C, the amount of impurities (e.g. the γ-butyrolactone) being formed increases.

In the present invention production process, it is important that the hydrogen pressure in the above hydrogenation reaction is set in the range of 0.1 to 2.0 MPa. This hydrogen pressure is more favorably set in the range of 0.3 to 1.8 MPa, still more favorably 0.5 to 1.5 MPa. In the case where the hydrogen pressure in the hydrogenation reaction is lower than 0.1 MPa, the reaction rate is very slow. In the case where the hydrogen pressure is higher than 2.0 MPa, there occur undesirable side reactions such as still more hydrogenation reaction.

Although there is no especial limitation on the conversion of the raw maleic anhydride in the above hydrogenation reaction in the present invention production process, yet it is favorably arranged that the conversion should not be less than 98 %. In the case where the above conversion is less than 98 %, what is called pre-distillation (of which the object is to remove low-boiling-point substances) is necessary, because, if the distillation is carried out without the pre-distillation, there is a possibility that: the succinic anhydride may be contaminated with the raw maleic anhydride in a large amount, or the coloring may progress with the passage of time when the succinic anhydride is left in a melted state. However, when the aforementioned pre-distillation is carried out, the low-boiling-point compounds (e.g. maleic anhydride) that are obtained as an initial distillate are separated, so that the yield of the succinic anhydride tends to be inevitably lowered.

In the present invention production process, it is favorable to intend the enhancement of the yield and conversion and the decrease of impurities by considering the reaction time along with various conditions (e.g. reaction temperature and hydrogen pressure) in the above hydrogenation reaction. There is no especial limitation on the reaction time. The reaction time will do if it is fitly arbitrarily set by also considering the combination with the above various conditions (e.g. reaction temperature and hydrogen pressure) so that the desired conversion of the maleic anhydride can be achieved. Generally, as to the above reaction temperature and hydrogen pressure, it is favorable that: the higher the temperature and the pressure are, the more the reaction time is shortened; and the lower the temperature and the pressure are, the more the reaction time is prolonged. Specifically, for example, the reaction time is favorably not shorter than 15 minutes. However, according to circumstances, there is also a case where the reaction time is favorably shorter than 10 minutes or not shorter than 300 minutes.

The above hydrogenation reaction is carried out in the presence of the catalyst. However, as to the catalyst, it is general and favorable to use a catalyst that can carry out hydrogenation across the carbon-carbon double bond portion of the cyclic-structure portion of the maleic anhydride. There is no especial limitation on such a catalyst. However, specifically, Raney catalysts and metal catalysts belonging to groups Ib and VIII which are transition metals are favorable.

The above Raney catalyst is a Raney including at least one of metals displaying the hydrogenation activity (e.g. Ni, Co, Cu, and Fe), and is a so-called skeletal catalyst having a skeleton. Accordingly, no support is necessary. In consideration of the costs, the Raney catalyst is favorably a Raney catalyst including only one or two of the above metal elements, more favorably including only one of the above metal elements. Above all, it is particularly favorable to use the Ni as the metal element.

The above metal catalysts belonging to groups Ib and VIII which are transition metals are, for example, metal catalysts including at least one of hydrogenation-active components such as Pd, Rh, Ru, Pt, Ir, Ni, Co, and Fe. Of these metal elements, Pd or Pt is more favorable, and Pd is particularly favorable. Although there is no especial limitation on the form of this metal catalyst, yet examples thereof include a metal powder, a slurry including the metal powder, and a form as supported on a support. Although there is no especial limitation on the above support, yet examples thereof include: aluminum oxide having various structures; SiO₂ having various structures; active carbon (carbon); mixtures of aluminum oxide and SiO₂; titanium dioxide; barium sulfate; and besides, a powder-form or molding-form of inert supports which are usually publicly known in the field of catalysts. In addition, in the case of the form as supported on the support, its shape is columnar or spherical favorably for withstanding such as temperature and pressure as used.

Although not especially limited, the amount of the catalyst as used in the above hydrogenation reaction is, for specific example, favorably in the range of 0.01 to 10 parts by weight, more favorably 0.1 to 5 parts by weight, still more favorably 0.2 to 3 parts by weight, per 100 parts by weight of the raw maleic anhydride.

In the present invention production process, the above hydrogenation reaction may be carried out either in a batch manner or in a flow (continuous) manner. Thus, there is no especial limitation.

In the present invention production process, the distillation of the resultant reaction product (crude succinic anhydride) is carried out after the above hydrogenation reaction.

In the present invention production process, it is important to carry out the aforementioned distillation in such a manner that the bottom temperature of the distillation column (which may hereinafter be referred to as the column-bottom temperature) will be within the temperature range of 125 to 200 °C (more favorably 130 to 190 °C, still more favorably 135 to 180 °C) by reducing the pressure. Thereby, there can be suppressed the formation of the dilactone which is a cause of the coloring. That is to say, as the column-bottom temperature of the distillation column becomes lower and/or as the heating time becomes shorter, the formation of the dilactone which is a cause of the coloring can be suppressed more and more effectively. If the column-bottom temperature of the distillation column is set in the aforementioned range, then it becomes possible to carry out the distillation at a temperature where the dilactone forms little and further to shorten the heating time. In the case where the column-bottom temperature of the distillation column is lower than 125 °C, such a temperature is not fit for the practical use. Its reason is as follows: the distillation rate of the succinic anhydride is so slow that it takes remarkably much time to carry out the distillation, and as a result, there are caused problems such that: the formation of the dilactone is easily caused by being heated for a long time, and besides, the clogging is caused by solidification of the recovered succinic anhydride. On the other hand, in the case where the column-bottom temperature of the distillation column is higher than 200 °C, the succinic anhydride becomes heated at such a high temperature that the formation of the dilactone increases when the distillation is carried out.

In the present invention production process, it is important to carry out the distillation in such a manner that the column-bottom temperature will be within the above temperature range. Hereupon, that the column-bottom temperature will be within the above range means that the column-bottom temperature will be maintained within the above range from the beginning to end of the flowing-out of the distillate. Incidentally, the column-bottom temperature refers to the main column-bottom temperature (positively controllable temperature), and it is enough that the substantial column-bottom temperature is maintained in the above range. That is to say, the column-bottom temperature may be outside the above range temporarily for a short time due to an operation as usually carried out during the distillation (e.g. switchover of pumps and exchange of distillate receivers).

In the present invention production process, the recovery ratio of the succinic anhydride in the distillation is favorably made to reach not less than 90 weight %, more favorably not less than 95 weight %, still more favorably not less than 99 weight %. In the case of carrying out the distillation, usually, as the recovery ratio of the succinic anhydride rises, in other words, as the amount of the distillated purified succinic anhydride increases, the column-bottom temperature of the distillation column tends to gradually rise. In the present invention production process, however, for suppressing the formation of the dilactone that is a coloring-causing substance, it is important to control the column-bottom temperature so as to be in the above temperature range all the time until the recovery ratio in the distillation reaches the above range.

The distillation may be carried out either in a batch manner or in a continuous manner. Thus, there is no especial limitation. In addition, the number of theoretical plates of the distillation column is favorably not smaller than 3, and the distillation column may be either a packed column or a plate column. There is no especial limitation thereto.

The reduced-pressure degree during the distillation will do if it is fitly set in such a manner that the column-bottom temperature of the distillation column will be within the aforementioned range. However, the reduced-pressure degree is favorably set in the range of 1.07 to 18.7 kPa. In addition, in the case of carrying out the distillation in the batch manner, the reduced-pressure degree may be lowered as the recovery ratio rises to raise the column-bottom temperature.

There is no especial limitation on the distillation apparatus as used for the distillation. The distillation apparatus will do if it is an apparatus equipped with at least such as: a distillation column, pressure-reducing and pressure-regulating means for controlling the column-bottom temperature of the distillation column, a heating means for dissolving and heating the crude succinic anhydride, and a receiver for recovering the distillated purified succinic anhydride.

If the distillation is such as batch type distillation, a specific procedure during the distillation is as follows.
(1) In the distillation apparatus, while the inside of the distillation column is set in a pressure-reduced state and while the internal pressure of the column-bottom portion is adjusted with such as a pressure-regulating valve, in such a manner that the column-bottom temperature of the distillation column will be within the above range, then the distillation of the melted crude succinic anhydride is carried out. Hereupon, it is favorable to set the distillation rate of the distillate in such a manner that the F factor will be in the range of 0.4 to 2.5. In addition, the reflux may be either carried out or not. However, in the case of carrying out the reflux, it is favorable to carry out the reflux in such a manner that the reflux ratio will be in the range of 0.5 to 5. Incidentally, it is favorable that the distillation is carried out under an atmosphere of an inert gas such as nitrogen. For example, it will do if air in the apparatus may be displaced with nitrogen after the charging of the crude succinic anhydride.
(2) The distillated succinic anhydride is recovered into the receiver. Hereupon, in the case where the recovered succinic anhydride includes low-boiling-point components such as unreacted raw material, it is favorable to remove, as an initial distillate, 0 to 10 weight % of the charged crude succinic anhydride, and further it is favorable to reuse the above initial distillate as the raw material for production of succinic anhydride.

As to the succinic anhydride as finally obtained, the present invention production process can suppress the content of the above dilactone (which is a by-product and impurity) and also the content of the γ-butyrolactone to low ones, and thereby can more enhance the quality of the succinic anhydride and also can reduce quality defects. Specifically, both of the γ-butyrolactone and the dilactone can be decreased in the concentration ranges as mentioned about the above present invention succinic anhydride.

The present invention production process can give the finally obtained succinic anhydride in a high yield. Although not especially limited, specifically, the succinic anhydride can be obtained in a yield of not less than 93 % relative to the raw maleic anhydride.

The succinic anhydride as obtained by the present invention production process is useful as a raw material for production of polyesters and, above all, suitable as a raw material for synthesis of aliphatic polyesters (particularly, biodegradable polyesters). Furthermore, the use of the succinic anhydride as obtained by the present invention production process is not limited thereto, but this succinic anhydride is, for example, useful also as a raw material for various chemical substances such as succinic acid, diesters of succinic acid, and succinimides.

The production process for a polyester, according to the present invention, comprises the step of carrying out a reaction between an acid component and an alcohol component to thereby produce the polyester, wherein the aforementioned present invention succinic anhydride is used as the acid component.

As to the acid component that is a raw material for the polyester, it is also possible to jointly use cyclic acid anhydrides (other than succinic anhydride) and/or dicarboxylic acids besides the aforementioned present invention succinic anhydride. Incidentally, the acid components other than the present invention succinic anhydride may be used either alone respectively or in combinations with each other.

Examples of the aforementioned cyclic acid anhydrides include maleic anhydride, itaconic anhydride, glutaric anhydride, adipic anhydride, and citraconic anhydride.

Examples of the aforementioned dicarboxylic acids include aliphatic dicarboxylic acids (e.g. succinic acid, adipic acid, suberic acid, sebacic acid, azelaic acid, decanedicarboxylic acid, and octadecanedicarboxylic acid) and aromatic dicarboxylic acids (e.g. terephthalic acid, isophthalic acid, o-phthalic acid, 1,4-naphthalenedicarboxylic acid, 2,3-naphthalenedicarboxylic acid, and 2,6-naphthalenedicarboxylic acid).

As to the alcohol component that is a raw material for the polyester, diols or cyclic ethers can be used.

Examples of the aforementioned diols include ethylene glycol, propylene glycol, 1,3-propanediol, 1,4-butanediol, neopentyl glycol, 1,5-pentanediol, 1,6-hexanediol, and decamethylene glycol. In addition, compounds in which portions of the glycols among the aforementioned exemplifying diols are polyoxyalkylene glycols can also be used as the diols. Examples thereof include polyoxyethylene glycol, polyoxypropylene glycol, polyoxytetramethylene glycol, and their copolymers. Incidentally, the diols may be used either alone respectively or in combinations with each other.

Examples of the aforementioned cyclic ethers include ethylene oxide, propylene oxide, butylene oxide, cyclohexene oxide, styrene oxide, epichlorohydrin, allyl glycidyl ether, phenyl glycidyl ether, tetrahydrofuran, oxepane, and 1,3-dioxolane. Incidentally, the cyclic ethers may be used either alone respectively or in combinations with each other.

The method for carrying out the reaction between the acid component and the alcohol component will do if it is fitly selected from among publicly known methods according to the kinds of the raw materials as used. Therefore, the method is not especially limited. For example, the method will do if there are adopted such as: 1) in the case of using the diol as the alcohol component, a method including the steps of causing the acid component (including the present invention succinic anhydride and, if necessary, further including the aforementioned cyclic acid anhydride and/or the aforementioned dicarboxylic acid) to react with the diol to thereby form a half ester, and thereafter carrying out polycondensation of the resultant half ester; and 2) in the case of using the cyclic ether as the alcohol component, a method including the step of carrying out ring-opening copolymerization of the acid component (including the present invention succinic anhydride and, if necessary, further including the aforementioned cyclic acid anhydride) and the cyclic ether.

There is no especial limitation on such as conditions of the half esterification and the polycondensation reaction in the aforementioned method 1), but the conditions will do if favorable conditions are fitly selected. For example, the entireties of the acid component and the diol may be initially charged in a lump and then caused to react together. Or either one of them may be initially charged and the other may be divided and then added thereto one by one with the progress of the reaction. The polycondensation reaction can be carried out by a conventional transesterification method or a conventional esterification method, or also by combination of both methods. In the case of the polycondensation reaction, general esterification catalysts and/or transesterification catalysts may be used. In addition, in the case of the polycondensation reaction, the polymerization degree may be adjusted by applying or reducing the pressure.

There is no especial limitation on such as conditions of the ring-opening copolymerization reaction in the aforementioned method 2), but the conditions will do if favorable conditions are fitly selected. For example, the ring-opening copolymerization reaction can be carried out by methods such as polymerization in a solvent and bulk polymerization with publicly known ring-opening-polymerization catalysts.

In the present invention production process for a polyester, the resultant low-molecular polyester may further be bonded to such as a small amount of polyfunctional isocyanate to form a polyester/polyurethane. Examples of the polyfunctional isocyanate include 2,4-tolylene diisocyanate, 2,6-tolylene diisocyanate, diphenylmethane diisocyanate, 1,5-naphthylene diisocyanate, xylylene diisocyanate, and hexamethylene diisocyanate. In addition, in the present invention production process for a polyester, the resultant low-molecular polyester may further be caused to react with such as a small amount of diphenyl carbonate to form a polyester/carbonate such as polybutylene succinate/carbonate.

In the present invention production process for a polyester, the succinic anhydride that is an essential raw material is a product as obtained by the aforementioned present invention production process. However, for omitting the re-heating step for the melting, it is favorable to provide the succinic anhydride to the production of the polyester, with the succinic anhydride left in the melted state without being solidified after the distillation. Specifically, for example, the distilled succinic anhydride may be placed directly into a reactor for the polyester production to carry out the production of the polyester. Or the distilled succinic anhydride may be preserved still in a melted state in a receptacle other than the reactor for the polyester production and, when the occasion demands, the preserved succinic anhydride may be transferred into the reactor for the polyester production to carry out the production of the polyester. Incidentally, the melted succinic anhydride in the reactor or in the receptacle for the preservation may beforehand be mixed with the acid component (other than the succinic anhydride) or the alcohol component which are used for the production of the polyester. For example, in the case of using the diol as the alcohol component, it is also possible that: the diol is beforehand charged into the reactor or the receptacle for the preservation, and then the melted succinic anhydride is dropwise added thereto; or the half esterification is beforehand carried out by dropwise adding the diol into the distilled succinic anhydride.

Specific examples of the polyester as obtained by the present invention production process for a polyester include polyethylene succinate and polybutylene succinate. In the case where the aforementioned cyclic acid anhydride and/or the aforementioned dicarboxylic acid is also used as an additional acid component, specific examples of the polyester include poly(butylene succinate/adipate), poly(butylene succinate/terephthalate), and poly(butylene succinate/adipate/terephthalate).

Although there is no especial limitation on the molecular weight of the polyester as obtained by the present invention production process for a polyester, yet the number-average molecular weight is favorably in the range of 10,000 to 200,000, more favorably 10,000 to 150,000, still more favorably 10,000 to 100,000.

In the present invention production process for a polyester, because the present invention succinic anhydride is used as a raw material, the resultant polyester is a polyester of a extremely reduced color. Specifically, the Yellow-Index (YI) of the polyester as obtained by the present invention production process for a polyester is favorably not more than 15, more favorably not more than 10, still more favorably not more than 7. In addition, the present invention production process for a polyester has the further advantage such as of enabling the shortening of the reaction time to thereby enhance the productivity, when compared with conventional production processes that involve the use of succinic acid.

### DETAILED DESCRIPTION OF THE PREFERRED EMBODIMENTS

Hereinafter, the present invention is more specifically illustrated by the following examples of some preferred embodiments. However, the present invention is not limited to them in any way. Incidentally, for convenience, the unit "part(s) by weight" may hereinafter be abbreviated simply to "part(s)".

### -Example 1 (production of succinic anhydride)-

An autoclave of 2 liters was charged with 1 kg of maleic anhydride and 4.0 g of an active-carbon-supported Pd (palladium) catalyst (Pd content: 5 weight %). Thereafter air in the autoclave was displaced with nitrogen, and then the autoclave was gradually heated to 140 °C under stirred conditions to thereby melt the maleic anhydride. Gases in the autoclave were exhausted, and thereafter the contents of the autoclave were stirred under a hydrogen pressure of 1.0 MPa for 300 minutes. Gases in the autoclave were exhausted again and then displaced with nitrogen. The resultant melted crude product in the autoclave was filtrated to thereby remove the catalyst. The conversion of the maleic anhydride as used was 99.5 %.

Incidentally, the conversion of the maleic anhydride was determined by dissolving the crude product (resultant from the hydrogenation reaction) into deuterated dimethyl sulfoxide in such a manner that the concentration of the crude product would be 10 weight %, and then measuring the resultant solution by ¹H-NMR (400 MHz). Also in the following Examples and Comparative Examples, the same method was used.

Subsequently, this crude product was melted and then distilled until the recovery ratio reached 99.5 weight % while the column bottom of a distillation column was maintained in the temperature range of 140 to 150 °C under a pressure of 2.7 to 4.0 kPa. By this distillation, there was obtained a product having a succinic anhydride content of 99.9 weight % and a γ-butyrolactone content of 400 ppm (this product may be hereinafter referred to as succinic anhydride (1)). There was seen no contamination of this succinic anhydride (1) with the maleic anhydride, the dilactone, or succinic acid. The yield of the succinic anhydride, as calculated from the amount of the raw maleic anhydride as used, was 95.0 %.

### -Example 2 (production of succinic anhydride)-

An autoclave of 2 liters was charged with 1 kg of maleic anhydride and 4.0 g of an active-carbon-supported Pd (palladium) catalyst (Pd content: 5 weight %). Thereafter air in the autoclave was displaced with nitrogen, and then the autoclave was gradually heated to 140 °C under stirred conditions to thereby melt the maleic anhydride. Gases in the autoclave were exhausted, and thereafter the contents of the autoclave were stirred under a hydrogen pressure of 1.0 MPa for 180 minutes. Gases in the autoclave were exhausted again and then displaced with nitrogen. The resultant melted crude product in the autoclave was filtrated to thereby remove the catalyst. The conversion of the maleic anhydride as used was 87.5 %.

Subsequently, this composition was distilled in the same way as of Example 1. As a result, there was obtained a product having a succinic anhydride content of 99.9 weight % and a γ-butyrolactone content of 50 ppm (this product may be hereinafter referred to as succinic anhydride (2)). There was seen no contamination of this succinic anhydride (2) with the maleic anhydride, the dilactone, or succinic acid. The yield of the succinic anhydride, as calculated from the amount of the raw maleic anhydride as used, was 83.0 %.

### -Comparative Example 1 (production of comparative succinic anhydride)-

An autoclave of 2 liters was charged with 1 kg of maleic anhydride and 4.0 g of an active-carbon-supported Pd (palladium) catalyst (Pd content: 5 weight %). Thereafter air in the autoclave was displaced with nitrogen, and then the autoclave was gradually heated to 140 °C under stirred conditions to thereby melt the maleic anhydride. Gases in the autoclave were exhausted, and thereafter the contents of the autoclave were stirred under a hydrogen pressure of 10 MPa for 300 minutes. Gases in the autoclave were exhausted again and then displaced with nitrogen. The resultant melted crude product in the autoclave was filtrated to thereby remove the catalyst. The conversion of the maleic anhydride as used was 99.8 %.

Subsequently, this composition was distilled in the same way as of Example 1. As a result, there was obtained a product having a succinic anhydride content of 99.9 weight % and a γ-butyrolactone content of 1,260 ppm (this product may be hereinafter referred to as comparative succinic anhydride (1)). There was seen no contamination of this comparative succinic anhydride (1) with the maleic anhydride, the dilactone, or succinic acid. The yield of the succinic anhydride, as calculated from the amount of the raw maleic anhydride as used, was 93.5 %.

### -Comparative Example 2 (production of comparative succinic anhydride)-

An autoclave of 2 liters was charged with 1 kg of maleic anhydride and 4.0 g of an active-carbon-supported Pd (palladium) catalyst (Pd content: 5 weight %). Thereafter air in the autoclave was displaced with nitrogen, and then the autoclave was gradually heated to 200 °C under stirred conditions to thereby melt the maleic anhydride. Gases in the autoclave were exhausted, and thereafter the contents of the autoclave were stirred under a hydrogen pressure of 4.0 MPa for 200 minutes. Gases in the autoclave were exhausted again and then displaced with nitrogen. The resultant melted crude product in the autoclave was filtrated to thereby remove the catalyst. The conversion of the maleic anhydride as used was 99.9 %.

Subsequently, this composition was distilled in the same way as of Example 1. As a result, there was obtained a product having a succinic anhydride content of 99.9 weight % and a γ-butyrolactone content of 1,500 ppm (this product may be hereinafter referred to as comparative succinic anhydride (2)). There was seen no contamination of this comparative succinic anhydride (2) with the maleic anhydride, the dilactone, or succinic acid. The yield of the succinic anhydride, as calculated from the amount of the raw maleic anhydride as used, was 95.2 %.

### -Comparative Example 3 (production of comparative succinic anhydride)-

There was carried out the same procedure as of Example 1 except to carry out the distillation while the column bottom of the distillation column was maintained in the temperature range of 210 to 220 °C under a pressure of 24 to 26 kPa. As a result, there was obtained a product having a succinic anhydride content of 99.5 weight %, a γ-butyrolactone content of 400 ppm, and a dilactone content of 1,000 ppm (this product may be hereinafter referred to as comparative succinic anhydride (3)). There was seen no contamination of this comparative succinic anhydride (3) with the maleic anhydride or succinic acid. The yield of the succinic anhydride, as calculated from the amount of the raw maleic anhydride as used, was 94.5 %.

### -Comparative Example 4 (production of comparative succinic anhydride)-

An autoclave of 2 liters was charged with 1 kg of maleic anhydride and 4.0 g of an active-carbon-supported Pd (palladium) catalyst (Pd content: 5 weight %). Thereafter air in the autoclave was displaced with nitrogen, and then the autoclave was gradually heated to 180 °C under stirred conditions to thereby melt the maleic anhydride. Gases in the autoclave were exhausted, and thereafter the contents of the autoclave were stirred under a hydrogen pressure of 1.5 MPa for 300 minutes. Gases in the autoclave were exhausted again and then displaced with nitrogen. The resultant melted crude product in the autoclave was filtrated to thereby remove the catalyst. The conversion of the maleic anhydride as used was 99.5 %.

Subsequently, this composition was distilled in the same way as of Example 1. As a result, there was obtained a product having a succinic anhydride content of 99.9 weight % and a γ-butyrolactone content of 1,350 ppm (this product may be hereinafter referred to as comparative succinic anhydride (4)). There was seen no contamination of this comparative succinic anhydride (4) with the maleic anhydride, the dilactone, or succinic acid. The yield of the succinic anhydride, as calculated from the amount of the raw maleic anhydride as used, was 95.0 %.

### -Comparative Example 5 (production of comparative succinic anhydride)-

There was carried out the same procedure as of Example 1 except to carry out the distillation while the column bottom of the distillation column was maintained under a pressure of 10 kPa. In this distillation,, the column-bottom temperature was 180 °C in the initial stage of the distillation (just after the distillate began to flow out), but began to rapidly rise around when the recovery ratio of the succinic anhydride had exceeded 98.0 weight %. Then, the column-bottom temperature reached 200 °C when the recovery ratio had reached 98.5 weight %. Furthermore, the distillation was continued until the recovery ratio reached 99.5 weight % (at that point of time, the column-bottom temperature had risen to 220 °C). As a result, there was obtained a product having a succinic anhydride content of 99.9 weight %, a γ-butyrolactone content of 400 ppm, and a dilactone content of 900 ppm (this product may be hereinafter referred to as comparative succinic anhydride (5)). There was seen no contamination of this comparative succinic anhydride (5) with the maleic anhydride or succinic acid. The yield of the succinic anhydride, as calculated from the amount of the raw maleic anhydride as used, was 94.5 %.

As to the succinic anhydrides (1) and (2) and the comparative succinic anhydrides (1) to (5) as obtained in the above ways, each of them was put into a SUS-316-made flask, and thereafter air in the flask was displaced with nitrogen, and thereafter the flask was heated to 150 °C to thereby melt the succinic anhydride. Subsequently, the succinic anhydride was sampled just after the succinic anhydride had melted and when the succinic anhydride had been retained at 150 °C for 7 hours since the succinic anhydride had melted. The melted samples as taken by the above sampling were poured into a disk-shaped receptacle having a diameter of 5 cm and then solidified, thus producing plate-shaped samples having a thickness of 5 mm. The Yellow-Indexes (YI) of these plate-shaped samples were measured with a color difference meter (product name: SE-2000, produced by Nippon Denshoku Kogyo Co., Ltd.). There were measured and calculated: a Yellow-Index (YI₀) of the plate-shaped sample as derived from the sample as taken just after the succinic anhydride had melted; and a value (ΔYI) as obtained by subtracting the above YI₀ from a Yellow-Index of the plate-shaped sample as derived from the sample as taken when 7 hours had passed since the succinic anhydride had melted (such a sample as has a larger value of ΔYI shows that its degree of coloring is larger). The results are listed in Table 1.

**[Table 1]**

| Sample name | YI₀ | ΔYI |
|---|---|---|
| Succinic anhydride (1) | -1.1 | 3.0 |
| Succinic anhydride (2) | -0 .8 | 2.9 |
| Comparative succinic anhydride (1) | 1.0 | 15.0 |
| Comparative succinic anhydride (2) | 0 9 | 10.5 |
| Comparative succinic anhydride (3) | 4.5 | 5 5 |
| Comparative succinic anhydride (4) | 1 .0 | 14.6 |
| Comparative succinic anhydride (5) | 3.7 | 5.2 |

As is understood from the results as listed in Table 1, the formation of the dilactone that is a by-product is suppressed by specifying the conditions in the distillation, so that the coloring (Yellow-Index) YI₀ of the distilled succinic anhydride is reduced. Furthermore, the formation of the γ-butyrolactone that is a by-product is suppressed by specifying the conditions in the hydrogenation reaction, so that the coloring (Yellow-Index) of the succinic anhydride itself as obtained is reduced, and further that there is remarkably reduced the coloring (Yellow-Index) in the case where this succinic anhydride is retained (preserved) in a melted state for a long time. Accordingly, it has been verified that: if the succinic anhydride is obtained by a process including the hydrogenation reaction of the raw maleic anhydride and the distillation of the resultant crude succinic anhydride, then the succinic anhydride which is obtained by specifying and controlling the respective conditions in the hydrogenation reaction and the distillation and in which the contents of the above two by-products are not higher than their respective specific ranges is always succinic anhydride of a very much reduced color both even (just) after the distillation and even in the case of having been preserved in a melted state for a long time.

### -Example 3 (production of polyester)-

A three-necked flask (reactor), as equipped with a thermometer, a stirring apparatus, a nitrogen-introducing tube, and a vigreux column, was charged with 120.08 parts of the succinic anhydride (1) and 76.61 parts of ethylene glycol and thereafter immersed into an oil bath. Subsequently, the contents of the flask were gradually heated to 185 °C under a stream of nitrogen under stirred conditions. Subsequently, polycondensation was carried out for 52 hours while the contents of the flask were maintained at 185 °C under a reduced-pressure degree of 100 kPa to normal pressure and while water being formed and excess ethylene glycol were distilled out. Thereafter, the reaction system was cooled down to ordinary temperature, thus obtaining an aliphatic polyester (which may hereinafter be referred to as aliphatic polyester (1)).

### -Example 4 (production of polyester)-

An aliphatic polyester (which may hereinafter be referred to as aliphatic polyester (2)) was obtained by carrying out the same procedure as of Example 3 except to use the succinic anhydride (2) instead of the succinic anhydride (1).

### -Comparative Examples 6 to 10 (production of comparative polyesters)-

Aliphatic polyesters of Comparative Examples 6 to 10 (which may hereinafter be referred to as comparative aliphatic polyesters (1) to (5)) were obtained by carrying out the same procedure as of Example 3 except to use the comparative succinic anhydrides (1) to (5) respectively instead of the succinic anhydride (1).

### -Example 5 (production of polyester)-

An autoclave was charged with 500 parts of the succinic anhydride (1), 3.68 parts of zirconyl octylate, and 250 parts of toluene, and then air in the autoclave was displaced with nitrogen. Subsequently, the autoclave was gradually heated to 130 °C under stirred conditions to thereby melt the succinic anhydride. Thereafter, while the pressure in the autoclave was maintained in the range of 0.40 to 0.83 MPa at the same temperature as the above, 231.1 parts of ethylene oxide was continuously introduced at an addition rate of 58 parts per hour over a period of 4.0 hours. After this introduction of the ethylene oxide, an aging reaction was carried out at 130 °C for 1.0 hour, and thereafter the reaction system was returned to ordinary temperature, thus obtaining a polymerized product. The toluene was removed from the polymerized product as obtained, thus obtaining an aliphatic polyester (which may hereinafter be referred to as aliphatic polyester (3)).

### -Example 6 (production of polyester)-

An aliphatic polyester (which may hereinafter be referred to as aliphatic polyester (4)) was obtained by carrying out the same procedure as of Example 5 except to use the succinic anhydride (2) instead of the succinic anhydride (1).

### -Comparative Examples 11 to 15 (production of comparative polyesters)-

Aliphatic polyesters of Comparative Examples 11 to 15 (which may hereinafter be referred to as comparative aliphatic polyesters (6) to (10)) were obtained by carrying out the same procedure as of Example 5 except to use the comparative succinic anhydrides (1) to (5) respectively instead of the succinic anhydride (1).

### -Example 7 (production of polyester)-

A three-necked flask (reactor), as equipped with a thermometer, a stirring apparatus, a nitrogen-introducing tube, and a vigreux column, was charged with 120.08 parts of the succinic anhydride (1) and 94.20 parts of 1,4-butanediol and thereafter immersed into an oil bath. Subsequently, the contents of the flask were gradually heated to 185 °C under a stream of nitrogen under stirred conditions. Subsequently, polycondensation was carried out for 35 hours while the contents of the flask were maintained at 185 °C under a reduced-pressure degree of 100 kPa to normal pressure and while water being formed and excess 1,4-butanediol were distilled out. Thereafter, the reaction system was cooled down to ordinary temperature, thus obtaining an aliphatic polyester (which may hereinafter be referred to as aliphatic polyester (5)).

### -Example 8 (production of polyester)-

An aliphatic polyester (which may hereinafter be referred to as aliphatic polyester (6)) was obtained by carrying out the same procedure as of Example 7 except to use the succinic anhydride (2) instead of the succinic anhydride (1).

### -Comparative Examples 16 to 20 (production of comparative polyesters)-

Aliphatic polyesters of Comparative Examples 16 to 20 (which may hereinafter be referred to as comparative aliphatic polyesters (11) to (15)) were obtained by carrying out the same procedure as of Example 7 except to use the comparative succinic anhydrides (1) to (5) respectively instead of the succinic anhydride (1).

As to the aliphatic polyesters (1) to (6) and the comparative aliphatic polyesters (1) to (15) as obtained in the above ways, each of them was melted at 130 °C under an atmosphere of nitrogen and thereafter solidified into the form of a plate having a thickness of 5 mm. This plate was cut with a nipper, thus obtaining pellets 3 mm square. These pellets were spread into a glass cell for measurement in such a manner that the thickness of the spread pellets would be 30 mm, and then their Yellow-Index (YI) was measured with a color difference meter (product name: SE-2000, produced by Nippon Denshoku Kogyo Co., Ltd.). The results are listed in Table 2.

In addition, as to the aliphatic polyesters (1) to (6) and the comparative aliphatic polyesters (1) to (15) as obtained in the above ways, their number-average molecular weights in terms of polystyrene were measured with a GPC apparatus (product name: HLC-8020, produced by Tosoh Corporation) under the following analysis conditions. The results are listed in Table 2.

### <Analysis conditions>:

Columns: Shodex K-802, K-803, K-804, K-805 (produced by Showa Denko K.K)
Eluent: chloroform
Flow rate: 1 ml/minute
Detector: Shodex RI
Column temperature: 40 °C
Concentration and quantity of sample: 1 weight % and 100 µl

**[Table 2]**

| Sample name | Succinic anhydride as used | Yellow-Index | Number-average molecular weight |
|---|---|---|---|
| Aliphatic polyester (1) | Succinic anhydride (1) | 3.5 | 35,000 |
| Aliphatic polyester (2) | Succinic anhydride (2) | 3.3 | 35,000 |
| Comparative aliphatic polyester (1) | Comparative succinic anhydride (1) | 18.2 | 34,000 |
| Comparative aliphatic polyester (2) | Comparative succinic anhydride (2) | 17.7 | 35,000 |
| Comparative aliphatic polyester (3) | Comparative succinic anhydride (3) | 15.5 | 35,000 |
| Comparative aliphatic polyester (4) | Comparative succinic anhydride (4) | 18.5 | 36,000 |
| Comparative aliphatic polyester (5) | Comparative succinic anhydride (5) | 15.6 | 34,000 |
| Aliphatic polyester (3) | Succinic anhydride (1) | 5.8 | 42,000 |
| Aliphatic polyester (4) | Succinic anhydride (2) | 5.2 | 42,000 |
| Comparative aliphatic polyester (6) | Comparative succinic anhydride (1) | 22.3 | 41,000 |
| Comparative aliphatic polyester (7) | Comparative succinic anhydride (2) | 21.8 | 42,000 |
| Comparative aliphatic polyester (8) | Comparative succinic anhydride (3) | 21.5 | 42,000 |
| Comparative aliphatic polyester (9) | Comparative succinic anhydride (4) | 22 5 | 41,000 |
| Comparative aliphatic polyester (10) | Comparative succinic anhydride (5) | 21.8 | 41,000 |
| Aliphatic polyester (5) | Succinic anhydride (1) | 3.8 | 38,000 |
| Aliphatic polyester (6) | Succinic anhydride (2) | 3.1 | 39,000 |
| Comparative aliphatic polyester (11) | Comparative succinic anhydride (1) | 17.9 | 39,000 |
| Comparative aliphatic polyester (12) | Comparative succinic anhydride (2) | 16.6 | 37,000 |
| Comparative aliphatic polyester (13) | Comparative succinic anhydride (3) | 15.3 | 37,000 |
| Comparative aliphatic polyester (14) | Comparative succinic anhydride (4) | 18.7 | 38,000 |
| Comparative aliphatic polyester (15) | Comparative succinic anhydride (5) | 15.8 | 37,000 |

### INDUSTRIAL APPLICATION

The present invention can provide: succinic anhydride that little becomes colored in a melted state; a production process for such succinic anhydride; and a production process for a polyester, by which there can be obtained a polyester of an extremely reduced color.

## Claims

1. Succinic anhydride, which is a raw material for production of polyesters and is obtained by a production process including the steps of: obtaining crude succinic anhydride by a hydrogenation reaction of maleic anhydride used as a raw material; and then purifying the crude succinic anhydride by distillation;
with the succinic anhydride being **characterized by** having a γ-butyrolactone content of not more than 800 ppm and a dilactone content of not more than 500 ppm.

2. A production process for succinic anhydride, which comprises the steps of: obtaining crude succinic anhydride by a hydrogenation reaction of maleic anhydride used as a raw material; and then purifying the crude succinic anhydride by distillation;
with the production process being **characterized in that**: the hydrogenation reaction is carried out in the presence of a catalyst in the temperature range of 120 to 160 °C under a hydrogen pressure of 0.1 to 2.0 MPa; and the distillation is carried out in such a manner that the bottom temperature will be within the temperature range of 125 to 200 °C by reducing the pressure.

3. A production process for a polyester, which comprises the step of carrying out a reaction between an acid component and an alcohol component to thereby produce the polyester; with the production process being **characterized in that** the succinic anhydride as recited in claim 1 is used as the acid component.
